# EUROPÄISCHE PATENTANMELDUNG

(11) **EP 0 645 357 A1**
(43) Veröffentlichungstag der Anmeldung: **29.03.1995**
(21) Anmeldenummer: 94114274.7
(22) Anmeldetag: 10.09.1994
(51) Int. Cl.: C07C 17/16, C07C 19/01

(54) **Verfahren zur Herstellung von Alkylchloriden**

(30) Priorität: 23.09.1993 DE 4332336
(71) Anmelder: BASF Aktiengesellschaft, D-67063 Ludwigshafen (DE)
(72) Erfinder: Ettl, Roland, Dr., D-67454 Hasslock (DE); Reuther, Wolfgang, Dr., D-69118 Heidelberg (DE)

(57) **Zusammenfassung**

Verfahren zur Herstellung von Alkylchloriden der allgemeinen Formel I 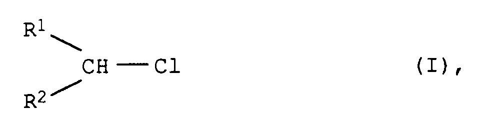
in der R¹ und R² für C₁- bis C₃₀-Alkyl, C₂- bis C₃₀-Alkenyl und C₂- bis C₃₀-Alkinyl, C₁- bis C₃₀-Hydroxyalkyl, C₃- bis C₁₂-Cycloalkyl, C₄- bis C₂₀-Alkyl-cycloalkyl, C₄- bis C₂₀-Cycloalkyl-alkyl, Aryl, C₇- bis C₂₀-Alkylaryl, C₇- bis C₂₀-Aralkyl steht, aus einem Alkohol der allgemeinen Formel II 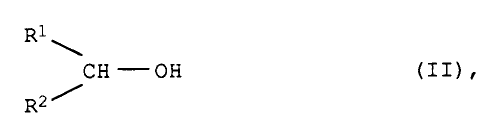
in der R¹ und R² die oben genannten Bedeutungen hat, und einem anorganischen Säurechlorid der allgemeinen Formel (III)

A-O-Cl₂ (III),

in der A Kohlenstoff oder Schwefel bedeutet, indem man die Umsetzung in Gegenwart eines Katalysator-Addukts aus III und N,N-disubstituiertem Formamid oder deren Hydrochloride der allgemeinen Formel IV 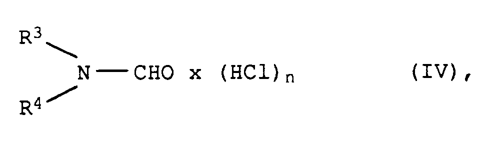
in der R³ und R⁴ C₁- bis C₈-Alkyl oder R³ und R⁴ gemeinsam eine C₄- oder C₅-Alkylenkette bedeuten, die gegebenenfalls durch ein Sauerstoffatom oder ein Stickstoffatom, das eine C₁- bis C₃-Alkylgruppe oder CHO trägt, unterbrochen sein kann und n O, 1 oder 2 bedeutet, bei Temperaturen von 20 bis 140°C durchführt.

## Beschreibung

Die vorliegende Erfindung betrifft ein neues und verbessertes Verfahren zur Herstellung von Alkylchloriden aus Alkoholen und Phosgen bzw. Thionylchlorid in einer Katalysatorphase, bestehend aus einem Addukt aus Phosgen bzw. Thionylchlorid und N,N-disubstituierten Formamid.

Alkylchloride werden meist aus den entsprechenden Alkoholen durch Chlorierung mit einem Halogenierungsreagens wie Phosgen, Thionylchlorid, Phosphor-(III)-chlorid und Phosphoroxychlorid hergestellt. Sie bilden wertvolle Zwischenprodukte für organische Synthesen, insbesondere von Pflanzenschutzmittel, Galvanohilfsmittel und Kunststoffvorprodukten.

In der DE-A-20 57 955 wird ein Verfahren zur Herstellung von 2-Chlor-3-butin beschrieben, dadurch gekennzeichnet, daß man bei tiefer Temperatur den Alkohol zunächst mit Phosgen zum Alkylchlorformiat umsetzt und dann in zweiter Stufe das Reaktionsgemisch bei Temperaturen oberhalb 60°C in Gegenwart von N,N-Dialkylformamiden zum Alkylchlorid zersetzt. Dieses Verfahren ist aufgrund der zweistufigen Reaktionsführung zeitaufwendig. Der in der ersten Stufe gebildete Chlorkohlensäureester ist sehr reaktiv und kann mit noch nicht umgesetztem Alkohol zum entsprechenden Dialkylcarbonat abreagieren. Das Alkylchlorid muß zur Abtrennung von Nebenprodukten destillativ gereinigt werden.

In der DE-A-36 11 419 bzw. DE-A-38 40 340 werden Verfahren zur Decarboxylierung von Alkylchlorformiaten in Gegenwart eines quartären Ammonium- oder Phosphoniumsalzes oder eines ternären Sulfoniumsalzes beschrieben. Die Umsetzung erfolgt bei hohen Temperaturen von bevorzugt 125 bis 140°C. Bei schwerflüchtigen Alkylchloriden verbleibt der Katalysator im Wertprodukt und kann nicht wiederverwendet werden. Bei den leichtflüchtigen Alkoholen ist zur Reinigung eine Kondensation bzw. eine Destillation angeschlossen. Die Umsetzung des Alkohols zum Alkylchlorid erfolgt in zwei Stufen und ist damit zeitaufwendig. Die Wiederverwendung des Katalysators ist nicht vorgesehen.

In der DE-A-41 16 365 wird ein Verfahren zur Herstellung von Alkylchloriden aus Alkoholen beschrieben, dadurch gekennzeichnet, daß man Alkohole mit Phosgen oder Thionylchlorid in Gegenwart eines Phosphinoxids als Katalysator umsetzt. Aufgrund der geringen Katalysatormenge von 0,005 bis 0,05 mol pro mol Hydroxylgruppe des Alkohols ist die Synthese zeitaufwendig; außerdem muß Phosgen mit 10 bis 20 % Überschuß, bezogen auf den Alkohol, eingesetzt werden. Anschließend muß das Wertprodukt durch Strippen von überschüssigem Phosgen befreit und durch Destillation gereinigt werden.

Der Erfindung lag nun die Aufgabe zugrunde, ein Verfahren zur Herstellung von Alkylchloriden zu finden, das den vorgenannten Nachteilen abhilft.

Demgemäß wurde ein neues und verbessertes Verfahren zur Herstellung von Alkylchloriden der allgemeinen Formel I 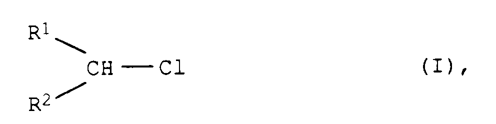
in der R¹ und R² für C₁- bis C₃₀-Alkyl, C₂- bis C₃₀-Alkenyl und C₂- bis C₃₀-Alkinyl, C₁- bis C₃₀-Hydroxyalkyl, C₃- bis C₁₂-Cycloalkyl, C₄- bis C₂₀-Alkyl-cycloalkyl, C₄- bis C₂₀-Cycloalkyl-alkyl, Aryl, C₇- bis C₂₀-Alkylaryl, C₇- bis C₂₀-Aralkyl steht, aus einem Alkohol der allgemeinen Formel II 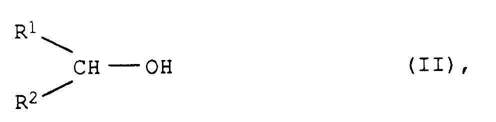
in der R¹ und R² die oben genannten Bedeutungen hat, und einem anorganischen Säurechlorid der allgemeinen Formel (III)

A-O-Cl₂ (III),

in der A Kohlenstoff oder Schwefel bedeutet, welches dadurch gekennzeichnet ist, daß man die Umsetzung in Gegenwart eines Katalysator-Addukts aus III und N,N-disubstituiertem Formamid oder deren Hydrochloride der allgemeinen Formel IV 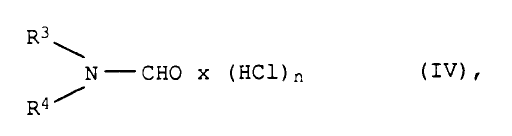
in der R³ und R⁴ C₁- bis C₈-Alkyl oder R³ und R⁴ gemeinsam eine C₄- oder C₅-Alkylenkette bedeuten, die gegebenenfalls durch ein Sauerstoffatom oder ein Stickstoffatom, das eine C₁- bis C₃-Alkylgruppe oder CHO trägt, unterbrochen sein kann und n O, 1 oder 2 bedeutet, bei Temperaturen von 20 bis 140°C durchführt.

Die Alkylchloride I sind nach folgender Methode erhältlich:
Man kann die Komponenten, den Alkohol II und das anorganische Säurechlorid III, stufenweise oder kontinuierlich bei Temperaturen von 20 bis 140°C vorzugsweise 45 bis 100°C, besonders bevorzugt bei 50 bis 80°C und Drucken von 0,01 bis 50 bar, vorzugsweise von 0,1 bis 5 bar, besonders bevorzugt bei Normaldruck (Atomsphärendruck) ca. 1 bar in einen mit dem Katalysator-Addukt aus Phosgen bzw. Thionylchlorid und N,N-disubstituierten Formamids IV gefüllten Reaktor leiten und zur Reaktion bringen.

Bei der stufenweise Reaktion kann man das Katalysator-Addukt durch Einleiten des anorganischen Säurechlorids III in das Formamid IV herstellen und anschließend einen Alkohol II durchleiten, bis das Katalysator-Addukt entladen ist und die Prozedur wiederholen.

Das Alkylchlorid I als Produkt trennt sich in aller Regel oberhalb der Katalysatorphase automatisch ab und kann in einem Vorratsgefäß aufgefangen werden und gegebenenfalls durch übliche Reinigungsmethoden wie Destillation nachgereinigt werden. Im allgemeinen ist die Produktqualität jedoch so, daß sich eine Reinigung erübrigt.

Bei der kontinuierlichen Variante kann man in einer bevorzugten Ausführungsform den Alkohol II und das anorganische Säurechlorid III in im wesentlichen äquimolaren Mengen des Chlorierungsreagens III zu den Hydroxylgruppen des Alkohols II in die Katalysatorphase einleiten.

Bei der Durchführung der kontinuierlichen Variante sollte bevorzugt die Dosierung der beiden Edukte Alkohol II und anorganisches Säurechlorid III in die Katalysatorphase räumlich getrennt erfolgen. Nach dem Durchgang der Edukte II und III durch die Katalysatorphase trennt sich in aller Regel das Alkylchlorid I als Produkt oberhalb der Katalysatorphase in einer Ruhezone automatisch ab. Es kann anschließend in einem Vorratsgefäß aufgefangen werden und gegebenenfalls durch übliche Reinigungsmethoden wie Destillation nachgereinigt werden. Im allgemeinen ist die Produktqualität jedoch so, daß sich eine Reinigung erübrigt.

Das Molverhältnis an Chlorierungsreagens III beträgt in der Regel 0,5 bis 10 mol, zweckmäßigerweise 1 bis 1,5 mol, vorzugsweise 1 bis 1,1 mol pro mol Hydroxylgruppe des Alkohols II.

Als anorganische Säurechloride eignen sich Thionylchlorid und bevorzugt Phosgen.

Die Phasentrennung kann in der Regel im Reaktor bei Temperaturen von 20 bis 100°C, vorzugsweise 30 bis 80°C, besonders 40 bis 70°C erfolgen.

Als Reaktoren eignen sich alle üblichen Reaktoren wie Rohrreaktoren, Schlangenreaktoren, Kessel, bevorzugt stehende Rohrreaktoren, denen von unten die Edukte zugeführt werden.

Der Reaktionsverlauf kann mit Hilfe von physikalischen Methoden kontrolliert werden, wie Dichte-, Leitfähigkeits- und Chlorid-Gehalts-Messungen.

Als Leitfähigkeitsmeßsonden eignen sich induktiv oder konduktiv messende Elektroden, bevorzugt induktiv messende. Die Leitfähigkeitsmeßsonden werden im Reaktor positioniert, bevorzugt in der Katalysatorphase. Die Katalysatorphase, also das Katalysator-Addukt aus Phosgen III bzw. Thionylchlorid IV und N,N-substituiertem Formamid x(HCl)ₙ (V) mit n = (0, 1, 2) kann aus einem Gemisch folgender Verbindungen bestehen: 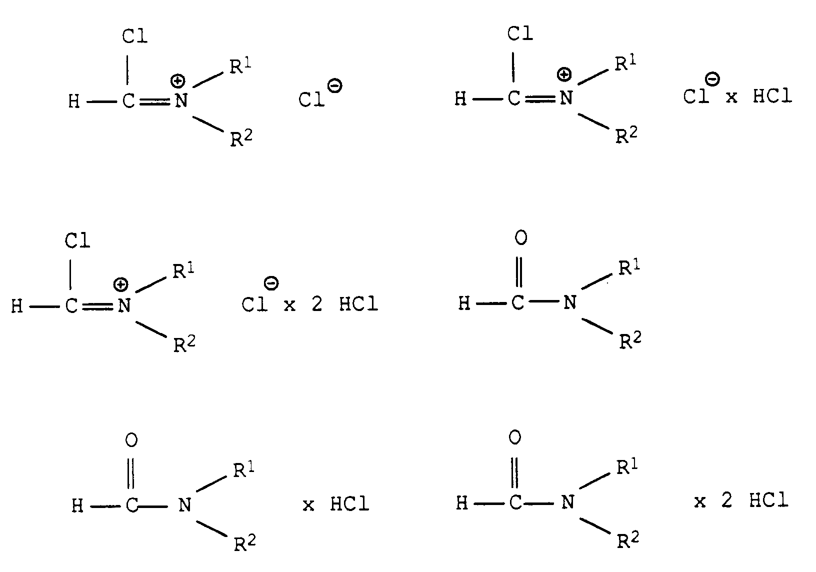
Der Beladungsgrad des Katalysators liegt in der Regel zwischen 0,1 und 95 mol-%, vorzugsweise 10 bis 80 mol-%, besonders bevorzugt bei 20 bis 70 mol-%.

Der Beladungsgrad des Katalysators kann kontinuierlich durch Messung der Dichte, der Viskosität, der Leitfähigkeit oder des Chlorid-Gehalts der im Trenngefäß anfallenden schwereren Phase bestimmt werden.

Man verwendet in der Regel 0,2 bis 10 mol, zweckmäßigerweise 0,5 bis 2,0 mol Katalysator pro mol Hydroxylgruppe des Alkohols, bevorzugt 0,8 bis 1,5 mol, besonders bevorzugt 0,9 bis 1,2 mol.

Da das Reaktionsabgas neben Kohlendioxid und Chlorwasserstoff nur unwesentliche Mengen anorganisches Säurechlorid enthält (0,0001 bis 0,1 %) ist eine Kondensation des Phosgens nicht erforderlich. Die Abgase können direkt der Abgaswäsche zugeführt werden. Zur Kühlung des Abgases sind deshalb nur solche Temperaturen notwendig, die das gebildete Wertprodukt möglichst vollständig kondensieren.

Dem Reaktionsgemisch kann, zur besseren Phasentrennung oder zur Umsetzung fester Alkohole, ein unter diesen Reaktionsbedingungen inertes Lösungsmittel zugesetzt werden, wie z.B. gesättigte aliphatische Kohlenwasserstoffe, Ether, Acetonitril, Benzol, Toluol oder Cyclohexan.

Ein besonderer Vorteil des Verfahrens besteht darin, daß auch feste Alkohole bei Temperaturen unterhalb ihrer Schmelztemperaturen ohne Lösungsmittel umgesetzt werden können.

Das Alkylchlorid fällt in hoher Ausbeute und in hoher Reinheit an. Es kann häufig ohne weitere Reinigung weiterverwendet werden. In einigen Fällen ist zur Erlangung hochreiner Produkte eine einfache Destillation erforderlich. Die Alkylchloride fallen nach der Umsetzung phosgenfrei an und alle Maßnahmen zur Entphosgenierung des rohen Alkylchlorids entfallen.

Das erfindungsgemäße Verfahren zur Herstellung von Alkylchloriden aus aliphatischen Alkoholen eignet sich vor allem für Monoalkohole sowie für Diole. Bevorzugte Alkohole sind z.B. 1,6-Hexandiol, 1,4-Butandiol, n-Octanol, n-Dodecanol, Oleylalkohol, 2-Ethylhexanol.

Als N,N-Dialkylformamide eignen sich Dimethyl-, Ethyl-methyl-, Methyl-n-propyl-, Methyl-iso-propyl-, Diethyl-, Ethyl-n-propyl, Ethyl-isopropyl-, Di-n-propyl-, n-Propyl-iso-propyl- und Di-iso-propyl-formamid, bevorzugt sind Dimethyl- und Diethylformamid.

Die Substituenten R¹, R², R³, R⁴ und des Indexes n haben folgende Bedeutungen:
- R¹ und R²: - C₁- bis C₃₀-Alkyl, bevorzugt C₁- bis C₂₀-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, n-Octyl, iso- Octyl, n-Nonyl, iso-Nonyl, n-Decyl, iso-Decyl, n-Undecyl, iso-Undecyl, n-Dodecyl und iso-Dodecyl, besonders bevorzugt C₂- bis C₄-Alkyl wie Ethyl, n-Propyl, iso-Propyl, n-Butyl, iso-Butyl, sec.-Butyl und tert.-Butyl,
- C₂- bis C₃₀-Alkenyl, bevorzugt C₂- bis C₈-Alkenyl wie Vinyl, Allyl, But-2-en-1-yl, But-4-en-1-yl, But-4-en-2-yl, Pent-2-en-1-yl und 2,2-Dimethyl-pent-1-en-1-yl, besonders bevorzugt C₂- bis C₆-Alkenyl wie Ethenyl, 1-Propenyl, 2-Propenyl, 1-Methyl-ethenyl, 1-Butenyl, 2-Butenyl, 3-Butenyl, 1-Methyl-1-propen-yl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl und 5-Hexenyl,
- C₂- bis C₃₀-Alkinyl, bevorzugt C₂- bis C₈-Alkinyl wie Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl und 5-Hexinyl,
- C₁- bis C₃₀-Hydroxyalkyl, bevorzugt C₁- bis C₈-Hydroxyalkyl, besonders bevorzugt C₁- bis C₄-Hydroxyalkyl wie Hydroxymethyl, 1-Hydroxyethyl, 2-Hydroxyethyl, 1-Hydroxy-n-propyl, 2-Hydroxy-n-propyl, 3-Hydroxy-n-propyl und 1-Hydroxy-methylethyl,
- C₃- bis C₁₂-Cycloalkyl, bevorzugt C₃- bis C₈-Cycloalkyl wie Cyclopropyl, Cyclobutyl, Cyclopentyl, Cyclohexyl, Cycloheptyl und Cyclooctyl, besonders bevorzugt Cyclopentyl, Cyclohexyl und Cyclooctyl,
- C₄- bis C₂₀-Cycloalkyl-alkyl, bevorzugt C₄- bis C₁₂-Cycloalkyl-alkyl wie Cyclopropyl-methyl, Cyclobutyl-methyl, Cyclopentyl-methyl, Cyclohexyl-methyl, Cyclopropyl-ethyl, Cyclobutyl-ethyl, Cyclopentyl-ethyl, Cyclohexyl-ethyl, Cyclopropyl-propyl, Cyclobutyl-propyl, Cyclopentyl-propyl und Cyclohexylpropyl, besonders bevorzugt Cyclopentyl-methyl, Cyclohexylmethyl, Cyclopentyl-ethyl und Cyclohexyl-ethyl,
- C₄- bis C₂₀-Alkyl-cycloalkyl, bevorzugt C₄- bis C₁₂-Alkyl-cyclo-alkyl wie 3-Methyl-cyclopentyl und 4-Methyl-cyclohexyl,
- Aryl, wie Phenyl, 1-Naphthyl, 2-Naphthyl, 1-Anthryl, 2-Anthryl und 9-Anthryl, bevorzugt Phenyl, 1-Naphthyl und 2-Naphthyl, besonders bevorzugt Phenyl,
- C₇- bis C₂₀-Aralkyl, bevorzugt C₇- bis C₁₂-Phenylalkyl wie Benzyl, 1-Phenethyl, 2-Phenethyl, 1-Phenyl-propyl, 2-Phenyl-propyl, 3-Phenyl-propyl, 1-Phenyl-butyl, 2-Phenyl-butyl, 3-Phenyl-butyl und 4-Phenyl-butyl, besonders bevorzugt Benzyl, 1-Phenethyl und 2-Phenethyl,
- C₇- bis C₂₀-Alkylaryl, bevorzugt C₇- bis C₁₂-Alkylphenyl wie 2-Methylphenyl, 3-Methylphenyl, 4-Methylphenyl, 2,4-Dimethylphenyl, 2,5-Dimethylphenyl, 2,6-Dimethylphenyl, 3,4-Dimethylphenyl, 3,5-Dimethylphenyl, 2,3,4-Trimethylphenyl, 2,3,5-Trimethylphenyl, 2,3,6-Trimethylphenyl, 2,4,6-Trimethylphenyl, 2-Ethylphenyl, 3-Ethylphenyl, 4-Ethylphenyl, 2-n-Propylphenyl, 3-n-Propylphenyl und 4-n-Propylphenyl,
- A: - Kohlenstoff oder Schwefel
- R³ und R⁴: - C₁- bis C₈-Alkyl, bevorzugt C₁- bis C₄-Alkyl wie Methyl, Ethyl, n-Propyl, iso-Propyl, n-Butyl, iso-Butyl, sec.-Butyl und tert.-Butyl, besonders bevorzugt Methyl und Ethyl oder
- R³ und R⁴ gemeinsam: - eine C₄- oder C₅-Alkylenkette gegebenenfalls durch ein Sauerstoffatom oder ein Stickstoffatom, das eine C₁- bis C₃-Alkylgruppe oder eine CHO-Gruppe trägt wie -(CH₂)₄-, -(CH₂)₅-, -(CH₂)₃-CH(CH₃)-, -(CH₂)₂-CH(CH₃)-CH₂)₂-, -(CH₂)₃-CH(CHO)-, -(CH₂)₂-O-(CH₂)₂- und -(CH₂)₂-N-(CH₂)₂-,
- n: - O, 1 oder 2.

### Beispiele

### Beladung des Katalysators

In einem Rührkolben werden 2 mol Dimethylformamid vorgelegt und durch Einleiten von Chlorwasserstoff in das entsprechende Hydrochlorid überführt. Anschließend werden zur Bildung des Katalysatoraddukts 4 mol Phosgen bei 60°C zudosiert.

### Beispiel 1

### Herstellung von Oleylchlorid

Zu dem beladenen Katalysator werden bei 60°C 2 mol Oleylalkohol (technische Ware; ca. 80 % ungesättigte Alkohole, ca. 20 % gesättigte Alkohole) innerhalb von 60 min zugesetzt. Zur Vervollständigung der Reaktion wird noch 15 min nachgerührt. Anschließend werden 2 mol Phosgen bei 60°C zudosiert. Das Produkt trennt sich oberhalb der Katalysatorphase ab und kann mit Hilfe eines Scheidetrichters isoliert werden.

Die Ausbeute beträgt 99 %. Das Produkt ist farblos und klar. Es kann kein Alkohol nachgewiesen werden.

### Beispiel 2

### Herstellung von 1,6-Dichlorhexan

Zu dem beladenen Katalysator werden bei 60°C 2 mol 1,6-Hexandiol innerhalb von 60 min zugesetzt. Zur Vervollständigung der Reaktion wird noch 15 min nachgerührt. Anschließend werden 2 mol Phosgen bei 60°C zudosiert. Das Produkt trennt sich oberhalb der Katalysatorphase ab und kann mit Hilfe eines Scheidetrichters isoliert werden.

Die Ausbeute beträgt 99 % mit einer Reinheit von 97,8 %. Das Produkt ist klar und farblos.

### Beispiel 3

### Herstellung von 1,4-Dichlorbutan

Zu dem beladenen Katalysator werden bei 60°C 2 mol 1,4-Butandiol innerhalb von 60 min zugesetzt. Zur Vervollständigung der Reaktion wird noch 15 min nachgerührt. Anschließend werden 2 mol Phosgen bei 60°C zudosiert. Das Produkt trennt sich oberhalb der Katalysatorphase ab und kann mit Hilfe eines Scheidetrichters isoliert werden.

Die Ausbeute beträgt 98 % mit einer Reinheit von 97,4 %. Das Produkt ist farblos und klar.

## Patentansprüche

1. Verfahren zur Herstellung von Alkylchloriden der allgemeinen Formel I 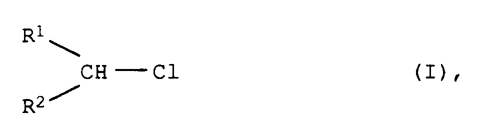 in der R¹ und R² für C₁- bis C₃₀-Alkyl, C₂- bis C₃₀-Alkenyl und C₂- bis C₃₀-Alkinyl, C₁- bis C₃₀-Hydroxyalkyl, C₃- bis C₁₂-Cycloalkyl, C₄- bis C₂₀-Alkyl-cycloalkyl, C₄- bis C₂₀-Cycloalkyl-alkyl, Aryl, C₇- bis C₂₀-Alkylaryl, C₇- bis C₂₀-Aralkyl steht, aus einem Alkohol der allgemeinen Formel II 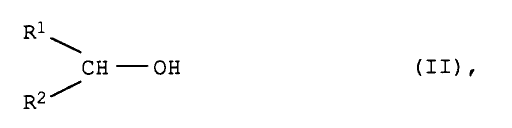 in der R¹ und R² die oben genannten Bedeutungen hat, und einem anorganischen Säurechlorid der allgemeinen Formel (III)
A-O-Cl₂ (III),
in der A Kohlenstoff oder Schwefel bedeutet, dadurch gekennzeichnet, daß man die Umsetzung in Gegenwart eines Katalysator-Addukts aus III und N,N-disubstituiertem Formamid oder deren Hydrochloride der allgemeinen Formel IV 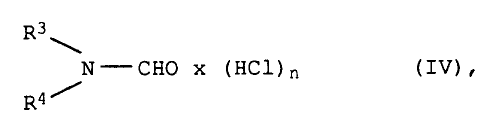 in der R³ und R⁴ C₁- bis C₈-Alkyl oder R³ und R⁴ gemeinsam eine C₄- oder C₅-Alkylenkette bedeuten, die gegebenenfalls durch ein Sauerstoffatom oder ein Stickstoffatom, das eine C₁- bis C₃-Alkylgruppe oder CHO trägt, unterbrochen sein kann und n O, 1 oder 2 bedeutet, bei Temperaturen von 20 bis 140°C durchführt.

2. Verfahren zur Herstellung von Alkylchloriden I nach Anspruch 1, dadurch gekennzeichnet, daß man die Umsetzung bei Temperaturen von 45 bis 100°C durchführt.

3. Verfahren zur Herstellung von Alkylchloriden I nach Anspruch 1, dadurch gekennzeichnet, daß man als N,N-disubstituiertes Formamid (IV) N,N-Dimethylformamid, Methyl-ethyl-formamid oder Diethylformamid einsetzt.

4. Verfahren zur Herstellung von Alkylchloriden I nach Anspruch 1, dadurch gekennzeichnet, daß man das N,N-disubstituierte Formamid (IV) zu 0,1 bis 95 mol-% mit einem anorganischen Säurechlorid (III) belädt.

5. Verfahren zur Herstellung von Alkylchloriden I nach Anspruch 1, dadurch gekennzeichnet, daß man die Umsetzung stufenweise durchführt.

6. Verfahren zur Herstellung von Alkylchloriden I nach Anspruch 1, dadurch gekennzeichnet, daß man die Umsetzung kontinuierlich durchführt.

7. Verfahren zur Herstellung von Alkylchloriden I nach Anspruch 1, dadurch gekennzeichnet, daß man 0,5 bis 2,0 mol Katalysatoraddukt pro mol Hydroxylgruppe des Alkohols (II) einsetzt.
